(19) **Europäisches Patentamt**
**European Patent Office**
**Office européen des brevets**

(11) **EP 3 988 084 B1**

(12) **EUROPEAN PATENT SPECIFICATION**

(45) Date of publication and mention
of the grant of the patent:
**03.12.2025 Bulletin 2025/49**

(21) Application number: **21204078.6**

(22) Date of filing: **21.10.2021**

(51) International Patent Classification (IPC):
***A61K 9/00*** (2006.01)

(52) Cooperative Patent Classification (CPC):
**A61K 9/1075; A61K 9/0095; A61K 31/05;
A61K 47/28**

(54) **CANNABINOID EMULSIONS**

CANNABINOID-EMULSIONEN

ÉMULSIONS DE CANNABINOÏDES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.10.2020 US 202063094701 P**

(43) Date of publication of application:
**27.04.2022 Bulletin 2022/17**

(73) Proprietor: **Corn Products Development Inc.
Westchester, IL 60154 (US)**

(72) Inventors:
• **HOLTHAUS, Derek**
**Bridgewater, NJ 08807 (US)**
• **NGUYEN, Quyen**
**Bridgewater, NJ 08807 (US)**
• **MAGNESS, Scott**
**Bridgewater, NJ 08807 (US)**

(74) Representative: **Novagraaf Group
Chemin de l'Echo 3
1213 Onex / Geneva (CH)**

(56) References cited:
**WO-A1-2020/018512     WO-A1-2020/243844
WO-A1-2021/046295     US-A1- 2021 177 013**

**Description**

The technology described in this specification relates to an emulsions comprising a cannabinoid and quillaja saponin, methods for producing such emulsions and uses of such emulsions.

Cannabinoid compounds are compounds found in cannabis. The best-known cannabinoids are tetrahydrocannabinol (THC) and cannabidiol (CBD). Other cannabinoids include cannabigerol (CBG), cannabichromene (CBC), cannabinol (CBN), cannabielsoin (CBE), iso-tetrahydrocannabinol (*iso*-THC), cannabicyclol (CBL), cannabicitran (CBT), cannabivarin (CBV), tetrahydrocannabivarin (THCV), THCP (tetrahydrocannabiphorol), cannabidivarin (CBDV), cannabichromevarin (CBCV), cannabigerovarin (CBGV), cannabigerol monomethyl ether (CBGM), tetrahydrocannabinolic acid (THCA), cannabidiolic acid (CBDA) and related compounds. Furthermore, cannabinoid compounds may include synthetic cannabinoids. These are generally molecules which are based on the structure of herbal cannabinoids.

Cannabidiol (CBD) oil extracted from hemp and marijuana (Cannabis sativa) is of particular interest due to its perceived health benefits which range from pain relief to anxiety suppression and beyond. Therefore, there is increased interest in incorporating CBD into foodstuffs to provide the aforementioned health benefits to consumers in an easily deliverable form (foodstuff). However, there are several key challenges associated with adding CBD oils into foodstuffs. First, CBD oils are not water soluble and thus cannot be homogenously incorporated into foodstuffs which are primarily water-based (e.g. beverages).

In the case of a beverage, if CBD oil were added into an existing formulation, the oil would float to the top and not evenly distributed throughout the beverage. This creates a significant challenge for dosing and quantification of CBD, as the top portion of the beverage would contain the entirety of the CBD while the rest of the beverage would not contain any. Also, CBD oil generally exhibits low oral bioavailability, as the digestive enzymes and other biological processes can only partially (and slowly) digest CBD oil and transport the CBD to the bloodstream. CBD also is very slow to reach the bloodstream upon oral administration, and thus there is a significant need to speed up the delivery.

The class cannabinoid further comprises other compounds which exhibit various effects. THC is for example the cannabinoid which is the primary psychoactive compound in cannabis.

Quillaia saponin is a known emulsifier. It is present in quillaja extract which can be extracted from the bark from the soap bark tree or *Quillaja saponaria*. The international patent application WO-2020018512 discloses a nano-emulsion composition comprising a cannabinoid oil, a dietically acceptable carrier oil, at least one surfactant, and water; wherein the amount of water is at least 1.15 times the amount of the combination of the cannabinoid oil, an dietically acceptable carrier oil and the surfactant (quillaja saponin, such as Q-Naturale 100). In the example 8 droplets having size around 200nm are generated resulting from the lower water to oil Mass Ratio of 1.9 : 1.

When using emulsions comprising cannabinoid in the disperse phase, it is believed that a smaller droplet size, and thus a larger surface area, may increase the digestive enzyme function and therefore increases oral bioavailability and time to onset, as well as reduces the required dosing of the cannabinoid to achieve a desired result.

In one aspect present technology provides an emulsion comprising (i) a continuous aqueous phase, (ii) a disperse oil phase comprising a cannabinoid, and (iii) an emulsifier comprising quillaja saponin, wherein said disperse oil phase has a median particle size (d50) of 200 nm or less, and wherein

    a. the weight fraction of the disperse oil phase in the emulsion is at least 35 wt.% based on the weight of the emulsion; and/or
    b. the weight ratio of water to disperse oil phase in the emulsion is less than 1.15:1.0, or is less than 1.1:1.0, or less than 1.0:1.0, wherein the weight ratio of quillaja saponin to said disperse oil phase is from 0.02:1 to 0.5:1.

Surprisingly, it has been found that when using quillaja saponin as emulsifier, a high oil load can be combined with a small particle size of the disperse phase comprising the cannabinoid.

In another aspect, the technology disclosed in this specification further provides a beverage comprising the emulsion as described in this specification.

In yet another aspect, the technology disclosed in this specification further provides a method of preparing a beverage, said method comprising incorporating and/or admixing the emulsion as described in this specification into said beverage. In another aspect, the technology disclosed in this specification further provides a beverage obtainable by this method.

In still another aspect, the technology disclosed in this specification further provides an emulsion or beverage according to the as described in this specification, for use as a medicament.

In another aspect, the technology disclosed in this specification provides an emulsion comprising (i) a continuous aqueous phase, (ii) a disperse oil phase comprising a cannabinoid, and (iii) an emulsifier comprising quillaja saponin, wherein said disperse oil phase has a median particle size (d50) of 200 nm or less, and wherein

    a. the weight fraction of the disperse oil phase in the emulsion is at least 35 wt.% based on the weight of the emulsion;

and/or

    b. the weight ratio of water to disperse oil phase in the emulsion is less than 1.15:1.

As will be understood by the skilled person, the emulsion is an oil-in-water emulsion, wherein oil phase droplets are dispersed within the aqueous continuous phase.

According to the some embodiments the weight fraction of the disperse oil phase in the emulsion is at least 35 wt.% based on the weight of the emulsion, and/or the weight ratio of water to disperse oil phase in the emulsion is of less than 1.15:1. The skilled person will understand that, as used herein, the weight of the disperse oil phase refers to the sum weight of the components present in the disperse oil phase, excluding emulsifiers present in the emulsion. The skilled person will further understand that, as used herein, the weight of water refers to the weight of the water, excluding emulsifiers and optional additives present in the emulsion.

In any embodiment the weight fraction of the disperse oil phase in the emulsion is at least 35 wt.% based on the weight of the emulsion. In any embodiment, the weight fraction of the disperse oil phase in the emulsion is at least 40 wt.% based on the weight of the emulsion, even more in at least 45 wt.% based on the weight of the emulsion. It was found that increasing the weight fraction of the disperse oil phase in the emulsion enables to decrease the particle size of the disperse oil phase even further.

There is no specific upper limit for the weight fraction of the disperse oil phase in the emulsion. The weight fraction of the disperse oil phase in the emulsion may for instance be 60 wt.% or less based on the weight of the emulsion, or 55 wt.% or less based on the weight of the emulsion.

In any embodiment, the weight ratio of water to disperse oil phase in the emulsion is less than 1.15:1.0. In any embodiment the weight ratio of water to disperse oil phase in the emulsion is less than 1.1:1.0, or less than 1.0:1.0. It was found that decreasing the weight ratio of water to disperse oil phase in the emulsion decreases the particle size of the disperse oil phase even further.

There is no specific lower limit for the weight ratio of water to disperse oil phase. The weight ratio of water to disperse oil phase in the emulsion may for instance be at least 0.8:1.0 based on the weight of the emulsion, or at least 0.9:1.0 based on the weight of the emulsion.

The weight ratio of water to disperse oil phase in the emulsion may for instance be from 0.8:1.0 to 1.15:1.0, or from 0.9:1.0 to 1.1:1.0, more or from 0.9:1.0 to 1.0:1.0.

The disperse oil phase has a median particle size (d50) of 200 nm or less. As used herein the median particle size (d50) is determined by a Malvern apparatus as described in the section "measurement methods". In any embodiment, the disperse oil phase has a median particle size (d50) from 50 nm to 180 nm, or from 100 nm to 150 nm.

Any suitable amount of quillaja saponin may be used. The weight ratio of quillaja saponin to disperse oil phase may for instance be from 0.02:1 to 0.5:1, or from 0.03:1 to 0.4:1, more or from 0.04:1 to 0.3:1.

The disperse oil phase may comprise any suitable cannabinoid. In any embodiment, the cannabinoid is selected from the group consisting of tetrahydrocannabinol (THC) and cannabidiol (CBD). In any embodiment the cannabinoid may also be THCA (tetrahydrocannabinolic acid), CBD (cannabidiol), CBDA (cannabidiolic acid), CBN (cannabinol), CBG (cannabigerol), CBC (cannabichromene), CBL (cannabicyclol), CBV (cannabivarin), THCV (tetrahydrocannabivarin), THCP (tetrahydrocannabiphorol), CBDV (cannabidivarin), CBCV (cannabichromevarin), CBGV (cannabigerovarin), CBGM (cannabigerol monomethyl ether), CBE (cannabielsoin), or CBT (cannabicitran).

The disperse oil phase may comprise a vegetable oil. The cannabinoid may be admixed with and/or dissolved in the vegetable oil. The vegetable oil may be any triglyceride oil extracted from seeds. Any suitable vegetable oil may be used, for instance a vegetable oil selected from the group consisting of medium chain triglyceride (MCT) oil, coconut oil, corn oil, cottonseed oil, olive oil, palm oil, peanut oil, rapeseed oil, safflower oil, sesame oil, soybean oil, sunflower oil, and canola oil. Generally, a vegetable oil has a density below that of water, hence below 1.0 g/ml.

It was found that the presence of a vegetable oil as disclosed hereinabove facilitates obtaining a stable emulsion in the event the cannabinoid has a low density. Without wishing to be bound by any scientific theory, it is believed that a vegetable oil having a density between the density of a cannabinoid having a low density and the density of water may assist to minimize the density difference between the disperse and continuous phase, thereby further enhancing the stability of the emulsion.

Based on the teaching provided herein, the skilled person can determine suitable ratios between the cannabinoid and the vegetable oil. The weight ratio of the cannabinoid and the vegetable oil may be between 1:0.1 and 1:9, for instance between 1:3 and 3:1. As used herein the weight of the cannabinoid refers to the sum weight of all cannabinoids which may be present in the disperse oil phase.

Quillaja saponin from any suitable source or any suitable quillaja tree extract may be used. An example of such an emulsifier is Q-NATURALE® 200V.

The emulsion may optionally contain any suitable optional additive, for instance a preservative. Exemplary additives which may be present in the emulsion include an acid, or an organic acid, for instance citric acid and/or ascorbic acid, potassium sorbate and/or sodium benzoate.

The emulsion can be prepared using methods known in the art. In any embodiment, this specification describes a method of making an emulsion comprising:

i. providing an aqueous phase comprising water and an emulsifier comprising quillaja saponin;
ii. providing an oil phase comprising an oil and a cannabinoid extract;
iii. mixing said aqueous phase and said oil phase to create a pre-emulsion; and
iv. homogenizing said pre-emulsion to obtain the emulsion.

In any embodiment, the said homogenizing comprises high pressure homogenization at a pressure of at least 130 bar, or between 240 and 2100 bar. In any embodiment, the homogenizing is affected in using a microfluidizer. In any embodiment, homogenizing is affected using at least 2 passes.

In another aspect, the technology disclosed in this specification further provides a beverage comprising the emulsion as described in this specification.

In another aspect, the technology disclosed in this specification further provides a method of preparing a beverage, said method comprising incorporating and/or admixing the emulsion as described in this specification into said beverage. In another aspect, the technology described in this specification further provides a beverage obtainable by this method.

In another aspect, the technology disclosed in this specification further provides an emulsion or beverage as described in this specification, for use as a medicament.

The technology disclosed in this specification can be better understood with reference to the following examples, which are not intended to limit the full scope of the invention.

## Measurement methods

*Turbiscan Stability Index (TSI)*

TSI is a parameter developed specially for formulators to rapidly compare and characterize the physical stability of various formulations and is measured using a Turbiscan Lab Expert (Formulaction) and software TurbiSoft-2.0.0.19. In any embodiment described in this specification, TSI is used to monitor the physical stability of the nanoemulsion concentrate. Any destabilization phenomenon that occurs in a sample will have an impact on the backscattering signal intensities over time. The formulation with the largest change in backscattering intensity is the least stable and has the highest TSI. The calculation of TSI is as follows:

$$TSI = \frac{\sum_h |scan_i(h) - scan_{i-1}(h)|}{H}$$

where the TSI calculation sums up the evolution of backscattered light at all measured position (h), based on a scan-to-scan difference, over total sample height (H).

Turbiscan vials (Formulaction) are filled 4 cm high with each emulsion concentrate and are measured for backscattering several times over a period of 21 days. At day 21, the TSI (Global) is recorded and the emulsion concentrates can be compared against each other for stability against destabilization phenomenon. Larger TSI values correspond to less stable emulsion concentrates.

*Particle size*

The median particle size (d50), as well as d10, d90, d[4,3] were measured using a particle size analyzer (Manufacturer: Malvern; Model: Mastersizer 2000).

## Examples

The embodiment of the technology described in this specification are numbered. Comparative examples are indicated using letters.

Example 1

*Emulsion preparation using high pressure homogenization.*

CBD nanoemulsions using quillaja extract as the emulsifier and hemp-based CBD isolate powder as the CBD source were prepared according to the formulations of Table 1.

Table 1: CBD emulsion formulations using quillaja extract as the emulsifier.

| Sample | Powder Quillaja Extract (%) | MCT Oil (%) | CBD Isolate (%) | Citric Acid (%) | Ascorbic Acid (%) | Potassium Sorbate (%) | Sodium Benzoate (%) | Water (%) |
|---|---|---|---|---|---|---|---|---|
| 1 | 15.00 | 30.00 | 20.00 | 0.20 | 0.20 | 0.10 | 0.10 | 34.40 |
| A | 15.00 | 3.00 | 2.00 | 0.20 | 0.20 | 0.10 | 0.10 | 79.40 |

The quillaja extract was in powdered form (QDP Ultra Organic from Desert King) and contains 65%-75% saponin. The CBD isolate powder ((>98% purity) was purchased from Treehouse Biotech (Longmont, CO).

The sodium benzoate and potassium sorbate were first dissolved in room temperature deionized water under stirring for 5 minutes. The quillaja extract was added to the solution and the mixture was stirred for 30 minutes. In a separate beaker, MCT oil was heated on a hot plate to 65°C. The CBD isolate powder was added to the MCT oil mixed until fully dissolved. The CBD oil solution was allowed to cool to room temperature.

A pre-emulsion was made by adding the oil phase into the aqueous phase under high shear mixing conditions: 10,000 rpm for 2 minutes in a Ross Model HSM-LCI-T.

The pre-emulsion was homogenized via high pressure homogenization at 344.7 bar (5000 psi) for 5 passes (1st stage =4500 psi/ 2nd stage =500 psi). The citric acid and ascorbic acid were added and the emulsion mixed an additional 5 minutes.

The particle size of the emulsion was immediately determined using a laser diffraction particle size analyzer (Malvern Mastersizer 2000). The median particle size ($d_{50}$), $d_{10}$, $d_{90}$, and d[4,3] were recorded.

*Beverage preparation*

Beverages were prepared from the obtained nanoemulsions, such that the CBD content was 25 mg per 355 g (~12 fl oz) of beverage. The beverages comprised 0.1 wt.% Sodium benzoate and 0.3 wt.% citric acid. Deionized water was added such that a total of 355 g beverage was obtained.

The citric acid and sodium benzoate were added to room temperature deionized water and mixed via magnetic stir bar for 5 minutes. The CBD nanoemulsion was added to the solution and lightly mixed. A 12 oz (~355 mL) bottle was filled with the solution and capped.

The bottle was stored horizontally at room temperature without manipulation for 21 days. After 21 days, the beverage was visually examined without manipulation for the presence of a white ring at the top of the beverage (creaming of the CBD emulsion). The beverage can also be examined for sedimentation.

The results of the trials are shown in Table 2 below.

Table 2: Particle size, beverage stability, and TSI results for emulsions made using quillaja extract as the emulsifier and CBD isolate powder as the CBD source.

| Sample | Pass | d10 (nm) | d50 (nm) | d90 (nm) | d[4,3] (nm) | Beverage Stability | TSI (Global) |
|---|---|---|---|---|---|---|---|
| 1 | 5 | 74 | 138 | 256 | 161 | Stable | 3.4 |
| A | 5 | 102 | 254 | 958 | 415 | Slight Ring | 4.6 |

Thus, it can be observed that the nanoemulsions, having a higher oil load, show smaller particle sizes than the comparative example and can produce beverages having improved stability.

Example 2

*Emulsion preparation using a microfluidizer*

CBD nanoemulsions were produced using a Microfluidizer (Microfluidics, Model: M-110EH) according to the formulations and processing instructions shown in Table 3. The microfluidizer has the capability to process emulsions at higher pressure and thus leads to more stable nanoemulsions and beverages. The interaction chamber used was the F12Y-H30Z. The quillaja extract was in liquid form as Q-Naturale 200 V from Ingredion Inc and contains 13-16 % saponins in an aqueous solution. The emulsions were sampled at various passes, and the final pass was used for beverage stability, turbidity, and TSI measurements.

Table 3: CBD emulsion formulations processed via microfluidization.

| Sample | LQE (%) | MCT Oil (%) | CBD Isolate (%) | Citric Acid (%) | Ascorbic Acid (%) | Potassium Sorbate (%) | Sodium Benzoate (%) | Water (%) | Passes |
|---|---|---|---|---|---|---|---|---|---|
| B | 35.0 | 6.0 | 4.0 | 0.2 | 0.2 | 0.1 | 0.1 | 54.4 | 1, 3, 5 |
| C | 35.0 | 12.0 | 8.0 | 0.2 | 0.2 | 0.1 | 0.1 | 44.4 | 1, 3, 5 |
| D | 35.0 | 18.0 | 12.0 | 0.2 | 0.2 | 0.1 | 0.1 | 34.4 | 1, 3, 5 |
| 2 | 35.0 | 24.0 | 16.0 | 0.2 | 0.2 | 0.1 | 0.1 | 24.4 | 1, 3, 5 |
| 3 | 35.0 | 30.0 | 20.0 | 0.2 | 0.2 | 0.1 | 0.1 | 14.4 | 1, 3, 5, 7 |
| 4 | 50.0 | 30.0 | 20.0 | - | - | - | - | - | 1, 3, 5 |

Pre-emulsions were made according to the previously described method.

The pre-emulsions were further processed via microfluidization (Manufacturer: Microfluidics) at 2068.4 bar (30000 psi) for the specified amount of passes. Citric acid and ascorbic acid were added to the solution and mixed an additional 5 minutes. The particle size of the emulsion was immediately tested using a laser diffraction particle size analyzer (Manufacturer: Malvern Mastersizer 2000) where the median particle size (d50), d10, d90, and d[4.3] were recorded.

*Beverage preparation*

Beverages were prepared according to the method of Example 1.

Beverage stability and TSI were measured according to the previously described methods.

Table 4: Particle size of emulsion, beverage stability, and TSI results for emulsions made via microfluidization.

| Sample | Pass | d10 (nm) | d50 (nm) | d90 (nm) | D[4.3] (nm) | Beverage Stability | TSI (Global) |
|---|---|---|---|---|---|---|---|
| B | 1 | 116 | 197.93 | 315.54 | 212.5 | - | - |
|  | 3 | 101.19 | 161.27 | 241.21 | 166.96 | - | - |
|  | 5 | 92.03 | 143.32 | 210.32 | 147.95 | Stable | 0.1 |
| C | 1 | 116.99 | 197.18 | 311.61 | 509.32 | - | - |
|  | 3 | 98.1 | 154.56 | 227.11 | 159.72 | - | - |
|  | 5 | 91.05 | 140.89 | 205.55 | 145.34 | Stable | 0.8 |
| D | 1 | 116.24 | 192.46 | 294.03 | 200.59 | - | - |
|  | 3 | 95.19 | 149.03 | 218.49 | 153.79 | - | - |
|  | 5 | 88.74 | 136.11 | 196.51 | 140.36 | Stable | 1.7 |
| 2 | 1 | 129.51 | 213.62 | 322.09 | 220.8 | - | - |
|  | 3 | 93.96 | 146.32 | 214.16 | 150.88 | - | - |
|  | 5 | 82.76 | 126.04 | 183.07 | 130.01 | Stable | 1.0 |
| 3 | 1 | 122.71 | 201.33 | 299.4 | 207.45 | - | - |
|  | 3 | 97.46 | 151.03 | 219.4 | 155.48 | - | - |
|  | 5 | 92.32 | 141.85 | 205.21 | 146.05 | - | - |
|  | 7 | 90.89 | 139.29 | 200.63 | 143.43 | Stable | 0.4 |

(continued)

| Sample | Pass | d10 (nm) | d50 (nm) | d90 (nm) | D[4.3] (nm) | Beverage Stability | TSI (Global) |
|---|---|---|---|---|---|---|---|
| 4 | 1 | 121.81 | 201.17 | 303.04 | 208.06 | - | - |
| | 3 | 88.51 | 135.41 | 195.52 | 139.61 | - | - |
| | 5 | 82.26 | 125.31 | 182.14 | 129.31 | Stable | 0.7 |

It can be observed that with increasing oil loads, the particle size, including d50 decreased.

**Claims**

1. An emulsion comprising (i) a continuous aqueous phase, (ii) a disperse oil phase comprising a cannabinoid, and (iii) an emulsifier comprising quillaja saponin, wherein said disperse oil phase has a median particle size (d50) of 200 nm or less, and wherein

   a. the weight fraction of the disperse oil phase in the emulsion is at least 35 wt.% based on the weight of the emulsion; and/or
   b. the weight ratio of water to disperse oil phase in the emulsion is less than 1.15:1.0, or is less than 1.1:1.0, or less than 1.0:1.0,
   wherein the weight ratio of quillaja saponin to said disperse oil phase is from 0.02:1 to 0.5:1.

2. The emulsion according to claim 1, wherein the weight fraction of the disperse oil phase in the emulsion is in a range selected from the group consisting of:

   a. at least 40 wt.% based on the weight of the emulsion, or at least 45 wt.% based on the weight of the emulsion;
   b. 60 wt.% or less based on the weight of the emulsion, or 55 wt.% or less based on the weight of the emulsion
   c. and mixtures thereof.

3. The emulsion according to any preceding claim, wherein the weight ratio of water to disperse oil phase in the emulsion is at least 0.8:1.0, to at least 0.9:1.0.

4. The emulsion according to any preceding claim, wherein said disperse oil phase has a median particle size (d50) from 50 nm to 180 nm, or from 100 nm to 150 nm.

5. The emulsion according to any preceding claim, wherein the weight ratio of quillaja saponin to said disperse oil phase is from 0.03:1 to 0.4:1, or from 0.04:1 to 0.3:1.

6. The emulsion according to any preceding claim, wherein said cannabinoid is

   a) one or more of tetrahydrocannabinol (THC) and cannabidiol (CBD) or is
   b) selected from the group consisting of tetrahydrocannabinol (THC) and cannabidiol (CBD) cannabigerol (CBG), cannabichromene (CBC), cannabinol (CBN), cannabielsoin (CBE), *iso*-tetrahydrocannabinol *(iso*-THC)*, cannabicyclol (CBL), cannabicitran (CBT), cannabivarin (CBV), tetrahydrocannabivarin (THCV), THCP (tetrahydrocannabiphorol), cannabidivarin (CBDV), cannabichromevarin (CBCV), cannabigerovarin (CBGV), cannabigerol monomethyl ether (CBGM), tetrahydrocannabinolic acid (THCA), cannabidiolic acid (CBDA) or mixtures thereof.

7. The emulsion according to any preceding claim, wherein, the disperse oil phase comprises a vegetable oil.

8. The emulsion according to preceding claim, wherein said vegetable oil is selected from the group consisting of medium chain triglyceride (MCT) oil, coconut oil, corn oil, cottonseed oil, olive oil, palm oil, peanut oil, rapeseed oil, safflower oil, sesame oil, soybean oil, sunflower oil and canola oil.

9. The emulsion according to any preceding claim, wherein the weight ratio of said cannabinoid to vegetable oil is between 1:0.1 and 1:9, for instance between 1:3 and 3:1.

**10.** A beverage comprising the emulsion according to any preceding claim.

**11.** A method of preparing a beverage, said method comprising incorporating and/or admixing the emulsion according to any preceding claim into said beverage.

**12.** Beverage obtainable by the method according to claim 11.

**13.** Emulsion or beverage according to any preceding claim, for use as a medicament.

**14.** Method for preparing an emulsion according to any one of claims 1 to 9, said method comprising:

i. providing an aqueous phase comprising water and an emulsifier comprising quillaja saponin,
ii. providing an oil phase comprising an oil and a cannabinoid extract,
iii. mixing said aqueous phase and said oil phase to create a pre-emulsion; and
iv. homogenizing said pre-emulsion to obtain the emulsion.


**Patentansprüche**

**1.** Emulsion, umfassend (i) eine kontinuierliche wässrige Phase, (ii) eine disperse Ölphase, umfassend ein Cannabinoid, und (iii) einen Emulgator, umfassend Quillaja Saponin, wobei die disperse Ölphase eine mittlere Partikelgröße (d50) von 200 nm oder weniger aufweist, und wobei

a. der Gewichtsanteil der dispersen Ölphase in der Emulsion mindestens 35 Gew.-% beträgt, bezogen auf das Gewicht der Emulsion; und/oder
b. das Gewichtsverhältnis von Wasser zu disperser Ölphase in der Emulsion geringer als 1,15 : 1,0 oder geringer als 1,1 : 1,0 oder geringer als 1,0 : 1,0 ist,
wobei das Gewichtsverhältnis von Quillaja Saponin zu der dispersen Ölphase von 0,02 : 1 bis 0,5 : 1 beträgt.

**2.** Emulsion nach Anspruch 1, wobei der Gewichtsanteil der dispersen Ölphase in der Emulsion in einem Bereich liegt, der aus der Gruppe ausgewählt ist, bestehend aus:

a. mindestens 40 Gew.-%, bezogen auf das Gewicht der Emulsion, oder mindestens 45 Gew.-%, bezogen auf das Gewicht der Emulsion;
b. 60 Gew.-% oder weniger, bezogen auf das Gewicht der Emulsion, oder 55 Gew.-% oder weniger, bezogen auf das Gewicht der Emulsion
c. und Mischungen davon.

**3.** Emulsion nach einem der vorstehenden Ansprüche, wobei das Gewichtsverhältnis von Wasser zu disperser Ölphase in der Emulsion mindestens 0,8 : 1,0 bis mindestens 0,9 : 1,0 beträgt.

**4.** Emulsion nach einem der vorstehenden Ansprüche, wobei die disperse Ölphase eine mittlere Partikelgröße (d50) von 50 nm bis 180 nm oder von 100 nm bis 150 nm aufweist.

**5.** Emulsion nach einem der vorstehenden Ansprüche, wobei das Gewichtsverhältnis von Quillaja Saponin zu der dispersen Ölphase von 0,03 : 1 bis 0,4 : 1 oder von 0.04 : 1 bis 0,3 : 1 beträgt.

**6.** Emulsion nach einem der vorstehenden Ansprüche, wobei das Cannabinoid ist

a) eines oder mehrere von Tetrahydrocannabinol (THC) und Cannabidiol (CBD) oder es ist
b) ausgewählt aus der Gruppe bestehend aus Tetrahydrocannabinol (THC) und Cannabidiol (CBD), Cannabigerol (CBG), Cannabichromen (CBC), Cannabinol (CBN), Cannabielsoin (CBE), Iso-Tetrahydrocannabinol (*Iso*-THC), Cannabicyclol (CBL), Cannabicitran (CBT), Cannabivarin (CBV), Tetrahydrocannabivarin (THCV), THCP (Tetrahydrocannabiphorol), Cannabidivarin (CBDV), Cannabichromevarin (CBCV), Cannabigerovarin (CBGV), Cannabigerolmonomethylether (CBGM), Tetrahydrocannabinolsäure (THCA), Cannabidiolsäure (CBDA) oder Mischungen davon.

**7.** Emulsion nach einem der vorstehenden Ansprüche, wobei die disperse Ölphase ein Pflanzenöl umfasst.

**8.** Emulsion nach dem vorstehenden Anspruch, wobei das Pflanzenöl aus der Gruppe ausgewählt ist, bestehend aus mittelkettigem Triglyceridöl (MCT-Öl), Kokosnussöl, Maisöl, Baumwollsamenöl, Olivenöl, Palmöl, Erdnussöl, Rapsöl, Färberdistelöl, Sesamöl, Sojabohnenöl, Sonnenblumenöl und Canolaöl.

**9.** Emulsion nach einem der vorstehenden Ansprüche, wobei das Gewichtsverhältnis des Cannabinoids zu dem Pflanzenöl zwischen 1 : 0,1 und 1 : 9, beispielsweise zwischen 1 : 3 und 3 : 1, liegt.

**10.** Getränk, umfassend die Emulsion nach einem der vorstehenden Ansprüche.

**11.** Verfahren zum Herstellen eines Getränks, das Verfahren umfassend ein Einarbeiten und/oder Zumischen der Emulsion nach einem der vorstehenden Ansprüche in das Getränk.

**12.** Getränk, erhältlich durch das Verfahren nach Anspruch 11.

**13.** Emulsion oder Getränk nach einem der vorstehenden Ansprüche zur Verwendung als ein Medikament.

**14.** Verfahren zum Herstellen einer Emulsion nach einem der Ansprüche 1 bis 9, das Verfahren umfassend:

i. Bereitstellen einer wässrigen Phase, umfassend Wasser und einen Emulgator, umfassend Quillaja Saponin,
ii. Bereitstellen einer Ölphase, umfassend ein Öl und einen Cannabinoidextrakt,
iii. Mischen der wässrigen Phase und der Ölphase, um eine Voremulsion zu erzeugen; und
iv. Homogenisieren der Voremulsion, um die Emulsion zu erhalten.

## Revendications

**1.** Émulsion comprenant (i) une phase aqueuse continue, (ii) une phase huileuse dispersée comprenant un cannabinoïde, et (iii) un émulsifiant comprenant de la saponine de quillaja, dans laquelle ladite phase huileuse dispersée a une taille de particule médiane (d50) de 200 nm ou moins, et dans laquelle

a. la fraction de poids de la phase huileuse dispersée dans l'émulsion est d'au moins 35 % en poids par rapport au poids de l'émulsion ; et/ou
b. le rapport en poids entre l'eau et la phase huileuse dispersée dans l'émulsion est inférieur à 1,15:1,0, ou inférieur à 1,1:1,0, ou inférieur à 1,0:1,0,
dans laquelle le rapport en poids entre la saponine de quillaja et ladite phase huileuse dispersée est de 0,02:1 à 0,5:1.

**2.** Émulsion selon la revendication 1, dans laquelle la fraction de poids de la phase huileuse dispersée dans l'émulsion se situe dans une plage choisie dans le groupe constitué par :

a. au moins 40 % en poids par rapport au poids de l'émulsion, ou au moins 45 % en poids par rapport au poids de l'émulsion ;
b. 60 % en poids ou moins par rapport au poids de l'émulsion, ou 55 % en poids ou moins par rapport au poids de l'émulsion
c. et des mélanges de ceux-ci.

**3.** Émulsion selon l'une quelconque des revendications précédentes, dans laquelle le rapport en poids entre l'eau et la phase d'huile dispersée dans l'émulsion est d'au moins 0,8:1,0, à au moins 0,9:1,0.

**4.** Émulsion selon l'une quelconque des revendications précédentes, dans laquelle ladite phase huileuse dispersée a une taille de particule médiane (d50) de 50 nm à 180 nm, ou de 100 nm à 150 nm.

**5.** Émulsion selon l'une quelconque revendication précédente, dans laquelle le rapport en poids entre la saponine de quillaja et ladite phase huileuse dispersée est de 0,03:1 à 0,4:1, ou de 0,04:1 à 0,3:1.

**6.** Émulsion selon l'une quelconque revendication précédente, dans laquelle ledit cannabinoïde est

a) un ou plusieurs parmi un tétrahydrocannabinol (THC) et un cannabidiol (CBD) ou est

b) choisi dans le groupe constitué de tétrahydrocannabinol (THC) et de cannabidiol (CBD) cannabigérol (CBG), cannabichromène (CBC), cannabinol (CBN), cannabielsoïne (CBE), iso-tétrahydrocannabinol (*iso*-THC), cannabicyclol (CBL), cannabicitran (CBT), cannabivarin (CBV), tétrahydrocannabivarine (THCV), THCP (tétrahydrocannabiphorol), cannabidivarine (CBDV), cannabichromévarine (CBCV), cannabigerovarine (CBGV), éther monométhylique de cannabigérol (CBGM), acide tétrahydrocannabinolique (THCA), acide cannabidiolique (CBDA) ou des mélanges de ceux-ci.

7. Émulsion selon l'une quelconque revendication précédente, dans laquelle la phase huileuse dispersée comprend une huile végétale.

8. Émulsion selon la revendication précédente, dans laquelle ladite huile végétale est choisie dans le groupe constitué par l'huile de triglycérides à chaîne moyenne (TCM), l'huile de coco, l'huile de maïs, l'huile de coton, l'huile d'olive, l'huile de palme, l'huile d'arachide, l'huile de colza, l'huile de carthame, l'huile de sésame, l'huile de soja, l'huile de tournesol et l'huile de canola.

9. Émulsion selon l'une quelconque revendication précédente, dans laquelle le rapport en poids entre ledit cannabinoïde et l'huile végétale est compris entre 1:0,1 et 1:9, par exemple entre 1:3 et 3:1.

10. Boisson comprenant l'émulsion selon l'une quelconque revendication précédente.

11. Procédé de préparation d'une boisson, ledit procédé comprenant l'incorporation et/ou le mélange de l'émulsion selon l'une quelconque revendication précédente dans ladite boisson.

12. Boisson pouvant être obtenue par le procédé selon la revendication 11.

13. Émulsion ou boisson selon l'une quelconque revendication précédente, pour une utilisation comme médicament.

14. Procédé de préparation d'une émulsion selon l'une quelconque des revendications 1 à 9, ledit procédé comprenant :

   i. la fourniture d'une phase aqueuse comprenant de l'eau et un émulsifiant comprenant de la saponine de quillaja,
   ii. la fourniture d'une phase huileuse comprenant une huile et un extrait de cannabinoïde,
   iii. le mélange de ladite phase aqueuse et de ladite phase huileuse pour créer une pré-émulsion ; et
   iv. l'homogénéisation de ladite pré-émulsion pour obtenir l'émulsion.

**EP 3 988 084 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020018512 A